# EUROPEAN PATENT APPLICATION

(11) **EP 4 325 202 A1**
(43) Date of publication of application: **21.02.2024**
(21) Application number: 22190403.0
(22) Date of filing: 15.08.2022
(51) Int. Cl.: G01N 17/02, G01N 33/38

(54) **METHOD AND DEVICE FOR ASSESSING CORROSION PHENOMENA**

(71) Applicant: ETH Zurich, 8092 Zurich (CH)
(72) Inventor: BIRCHER, Lukas, 6362 Stansstad (CH); ANGST, Ueli, 5436 Würenlos (CH); PFÄNDLER, Patrick, 8046 Zürich (CH)

(57) **Abstract**

The invention relates to a method for assessing corrosion phenomena on at least one metal element (1) of a concrete structure (2) covered at least in regions with a material (3) that is electrolytically conductive or can be made electrolytically conductive, in which an electrolyte volume (4) is positioned within the material (3), an electrode (5) is arranged in the electrolyte volume (4) so that the electrode (5) and the electrolyte are in electrically conductive connection, and the electrolyte volume (4) is brought into electrically conductive contact with the material (3), and an electrochemical measurement indicative of a state of corrosion on the metal element (1) is carried out.

The invention also relates to a device (11) for assessing corrosion phenomena on at least one steel element (1) of a concrete structure (2) covered at least in regions with a material (3) that is electrolytically conductive or can be made electrolytically conductive.

## Description

The present invention relates to a method for assessing corrosion phenomena as well as a device for assessing corrosion phenomena

Steel-reinforced concrete is one of the most used materials in the building industry. While it is generally durable, corrosion of the embedded reinforcing steel elements is among the most frequent causes for premature degradation. This has severe consequences for safety, economy, and environment. Detection of corrosion ahead of structural damage is thus of large importance. While various techniques for corrosion inspection exist, it is a major problem that some structural members cannot be inspected, simply because of a lack of accessibility. An example that has received increasing attention in engineering is the backside of cantilever retaining walls, which were found to show an increased risk for local corrosion, but can hardly be inspected with any available technology. In particular old or low-quality steel-reinforced concrete structures such as cantilever retaining walls therefore represent sources of danger, as failure or collapse due to corrosion phenomena of the steel elements forming the backbones of such cantilever retaining walls can cause fatal accidents and high repair costs. However, this problem is not limited to cantilever retaining walls as similar inspection challenges are also met in other cases where a metallic structure is buried in the ground, such as foundations, piles, reservoirs, storage tank bottoms, or pipes.

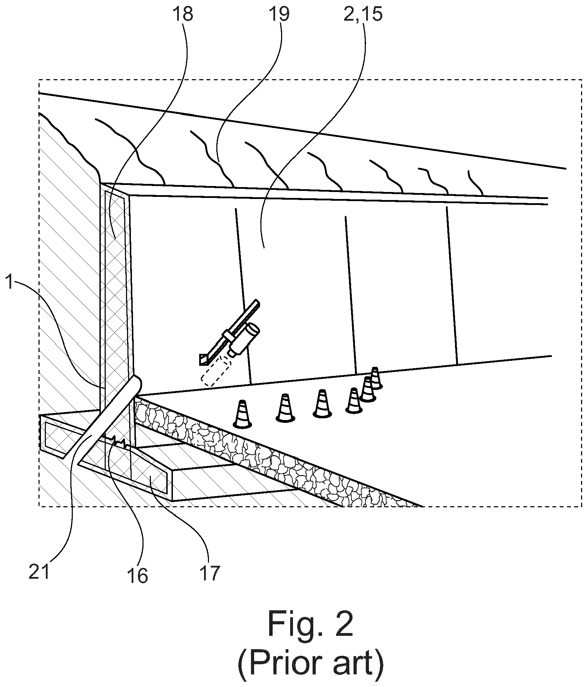

To tackle this problem, core drillings are commonly used to locally extract samples of the reinforcing steel element by drilling a hole into the concrete structure, so as to access the steel element, cf. Fig. 2. However, this procedure represents an invasive action that itself negatively affects the concrete structure and its stability. Moreover, this allows to only locally extract and analyze samples, such that possibly dangerous corrosion phenomena on the steel element in the proximity of or generally outside the drill holes selected for extraction of samples cannot be assessed.

As a largely non-destructive procedure, potential field measurements have gained importance for assessing the state of corrosion of steel elements in concrete structures. For example, WO 2019084694 A1 discloses a method wherein a first electrical port of a voltmeter is electrically connected to a reference electrode, which is brought into electrical contact with a surface of a concrete structure embedding a steel element by drilling a hole through an asphalt overlay covering the concrete structure and filling the hole with a conductive solution, until electrical contact with the electrode located on the asphalt overlay is reached. A second electrical port of the voltmeter is electrically connected to the steel element. By measuring the voltage between the steel element and the reference electrode, state of the corrosion of the steel element may be assessed at the location of the reference electrode. However, the reinforcing steel elements of underground concrete structures are often buried by large layers of material, such as soil, such that this method regularly requires excavation of material covering the concrete structure, so as to get close enough to detect the potential field of the steel element, as the potential field decays with the distance from the steel element.

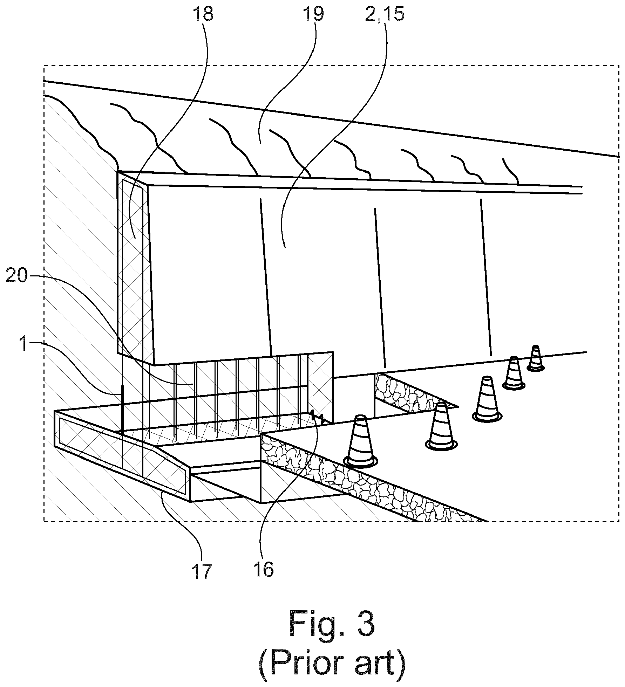

To this end, parts of the concrete structure or structures or material covering the concrete structures are often removed, for example by high pressure water jetting, so as to expose the steel element for visual inspection, cf. Fig. 3, However, this procedure also represents a strong intervention into the structure of the concrete structure, which itself harms its stability. At the same time, while larger inspection windows may be opened by removing more concrete - at the price of a loss of stability - the assessment of the state of corrosion on the steel element reached by this procedure is still constricted to the extent of the inspection window. The state of corrosion outside the inspection window thus remains unknown, such that, potentially dangerous corrosion phenomena outside of the inspection window selected for inspection may remain hidden.

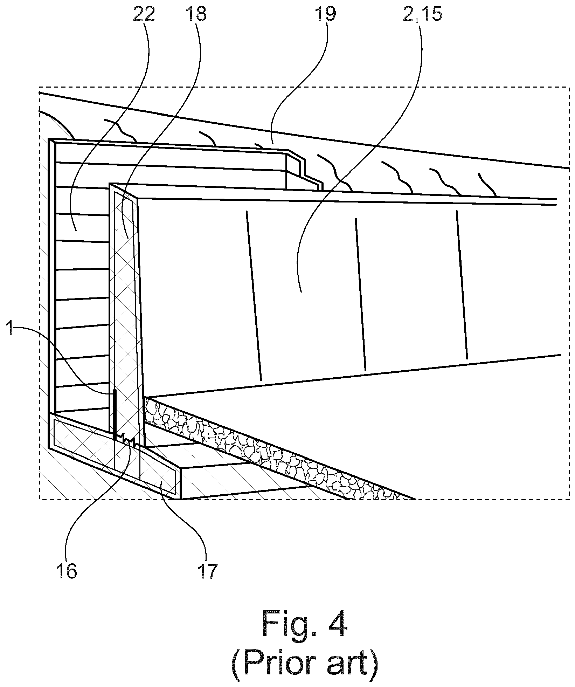

Yet another known method for assessing corrosion phenomena, particularly for partially buried concrete structures, provides that an inspection shaft is excavated so as to access the steel element, cf. Fig. 4 or to get close enough to perform a potential field measurement. Depending on the accessibility of the steel element due to its arrangement inside the concrete structure, this method may require less or no removal of concrete as in the previously described procedure, but still, the assessment of the state of corrosion remains constricted to the region of the excavated inspection shaft. At the same time, the excavation of large amounts of material covering the concrete structure, such as soil, is usually complex and expensive.

As such, none of the above-described methods allows an economical inspection for instance over the entire length of a retaining wall, as the costs related to the excavation, or removal of material would be on the order of 10 % of the new construction costs according to the Swiss Federal Office for Road Infrastructure and Individual Road Traffic. Thus, only random local inspections can be performed and there is a risk that critical corrosion phenomena will not be detected.

Based on this, it is subject of the present invention to provide a method and a device that permits to assess the state of corrosion on at least one metal element of a concrete structure in a largely non-destructive manner without the necessity to excavate large amounts of material covering the concrete structure.

This task is solved by a method for assessing corrosion phenomena with the features of claim 1 as well as a device for assessing corrosion phenomena with the features of claim 10.

Advantageous embodiments of the invention are given in the subclaims 2 to 9 of the method for assessing corrosion phenomena as well as the subclaims 11 to 15 of the device for assessing corrosion phenomena, which are described in the following.

A first aspect of the invention relates to a method for assessing corrosion phenomena on at least one metal element of a concrete structure covered at least in regions with a material that is electrolytically conductive or can be made electrolytically conductive. According to this method, an electrolyte volume is positioned within the material, an electrode is arranged in the electrolyte volume so that the electrode and the electrolyte are in electrically conductive connection, and the electrolyte volume is brought into electrically conductive contact with the material, and an electrochemical measurement indicative of a state of corrosion on the metal element is carried out.

As such, this method allows to assess corrosion phenomena of metal elements of concrete structures even if they are covered with the material, such that there is no necessity to excavate or remove large amounts of the material, which may for example comprise or be soil.

According to the invention, the material can be an electrolytically conductive material, such as for example soil.

Alternatively, the material can be made electrolytically conductive by introducing an electrolyte into the material. As such, the method according to the invention can also be applied to dry and/or porous materials such as for example gravel or scree by making the material electrolytically conductive, for example by watering the material before carrying out the method according to the invention.

Since corrosion phenomena on metal elements in concrete structures are usually caused by electrochemical processes, they can be detected using electrochemical measurements or methods. For example, the electrochemical measurement according to the invention comprises at least one of the following: potential measurements, particularly potential field measurements; electrochemical impedance measurements; galvanostatic- or potentiostatic polarization- as well as galvanodynamic- or potentiodynamic polarization measurements, for instance linear polarization resistance measurements; Harmonic analysis or Faradaic rectification measurements as well as other electrochemical methods in general.

As an underlying process, for example ingress of moisture and/or chloride through cracks, honeycombs or gaps of the concrete structure causes regions of the metal element to become subject to corrosion, wherein the affected regions turn anodic. In turn, regions that are not subject to corrosion, represent cathodic regions. As a consequence, a spatial, three-dimensional electrochemical environment, e.g. potential field, around regions of corrosion of the metal element is indicative of the state of corrosion of the metal element. Particularly, the metal element is a steel element. The electrochemical measurement according to the invention allows to obtain, particularly to measure a physical quantity related to the electrochemical environment, thereby representing information indicative of the state and location of corrosion in the area of the metal element. While the potential field measurement takes advantage of the intrinsic potential field generated by the regions subject to corrosion themselves, the potential field and/or electrical quantities related to the corrosion state and/or the electrochemical environment may be additionally excited in the electrochemical measurements, so as to increase the accuracy of the measurement or to obtain additional information.

In a first example for a potential field measurement indicative of the state of corrosion, an electrical potential difference or a voltage between the electrode and the metal element is measured, for example using a voltmeter. To this end, a first electrical port of the voltmeter is electrically connected to the metal element, which may require a single local bore through the material and the concrete structure, depending on the arrangement of the metal element in the concrete structure and the material. A second electrical port of the voltmeter is electrically connected to the electrode, such that the potential difference or voltage between the electrode and the metal element may be determined at the corresponding location of the electrode. A value of the potential difference or voltage measured at this location corresponds to or is indicative of a value of the spatial potential field at this location. In particular, more negative measured potential values or higher potential gradients point to a more critical state of corrosion and less negative measured potential values point to a less critical state of corrosion.

In a second example for a potential field measurement indicative of the state of corrosion, the electrical connection to the steel element is replaced with an electrical connection to a reference electrode. The reference electrode may be a stationary reference electrode which is kept at a fixed location. By measuring the potential difference or voltage between the electrode and the reference electrode, the value of the potential field at the location of the electrode may be determined. Particularly, the electrode may be moved to different locations and multiple potential field measurements may be carried out, such that the potential field is determined at the different locations, improving the resolution of the spatial potential field.

In a third example for a potential field measurement indicative of the state of corrosion, the electrode is electrically connected to a reference electrode as in the second example, wherein the electrode and the reference electrode are moved together while keeping a fixed distance between the electrode and the reference electrode. In particular, in this third example, a gradient of the measured potential difference or voltage between the electrode and the reference electrode is determined.

Alternatively or additionally, the state of corrosion may be assessed by means of a linear polarization resistance measurement.

For example, the linear polarization resistance is determined in a potentiostatic sweep measurement/method. Here, in a three-electrode setup a constant predetermined electrical potential is applied to the metal element serving as a working electrode, and the resulting current flow between the counter electrode and the metal element is measured under stationary conditions, that is for large timescales. This measurement can also be performed in a two electrodes configuration setup, where the counter and reference electrode coincide.

The polarization resistance may also be determined by an impedance measurement. Here, in a three-electrode setup an alternating voltage as a function of its frequency (f) is applied between the metal element and the reference electrode with a predetermined amplitude and the resulting current flow between the counter electrode and the metal element is measured in the frequency space, wherein an impedance spectrum is obtained. The polarization resistance may then be determined by extrapolating the impedance spectrum towards f → 0. This measurement may also be performed in a two-electrode setup, where the counter and reference electrode coincide.

Moreover, the polarization resistance may be determined in a galvanostatic pulse measurement. Here, in a three-electrode setup, a current pulse of predetermined time and amplitude is applied between metal element and the counter electrode and the resulting potential difference between the reference electrode and the metal element is measured as a function of time. The polarization resistance may then be determined by analyzing the response in the time domain with equivalent circuits. This measurement may also be performed in a two-electrode setup, where the counter and reference electrode coincide.

Particularly, a small polarization resistance corresponds to a more critical state of corrosion and a large polarization resistance corresponds to a less critical state of corrosion.

Alternatively or additionally, the state of corrosion may be assessed by means of a Faradaic rectification measurement. Here, an external alternating voltage is applied between the electrode and the metal element and the resulting change in direct current measured gives information on the corrosion state.

Optionally, to improve the accuracy of the electrochemical measurement over the measurement on the surface, bores are drilled into the material, so as to bring the electrode in the electrolyte closer to the metal element, while keeping the electrolyte reservoir spaced from the concrete structure via the material. The bores may for example extend vertically, that is, along the direction of gravity, or horizontally, that is, perpendicular to the direction of gravity.

According to an embodiment, the electrolyte volume is placed in a container for receiving the electrolyte volume in or next to the material, realizing an electrolyte reservoir. As such, a good electrically conductive connection between the electrolyte volume and the electrode is provided, once the electrolyte volume is placed in the container.

In an embodiment, the container is at least a part of a pipe or is a pipe. The container may thus be formed by or in existing pipes or pipe systems, such as drainage lines. This is in particular advantageous for assessment of metal elements embedded in cantilever retaining walls, which are often neighbored by such drainage lines. As such, the electrode in the electrolyte volume may be brought sufficiently close to the metal element to perform an electrochemical measurement without the necessity to excavate or remove large amounts of material by arranging it in the pipe. In particular, the electrode is arranged in a distance of less than 1 m to the metal element.

For example, the pipe may run vertically or horizontally. Further, the pipe may be closed at one end, restricting the reservoir on this side.

In yet another embodiment, the electrolyte is pumped out of the electrolyte volume and into the material with an electrolyte supply unit, particularly a pump unit, such that the electrode in the electrolyte volume is brought into and kept in electrically conductive contact with the material. This embodiment is particularly advantageous to maintain the electrically conductive contact despite a drain or seeping of the electrolyte out of the area between the electrolyte volume and the concrete structure, which occurs particularly if the material between the electrolyte volume and the concrete structure is porous such as gravel or if the material tends to absorb the electrolyte, as for example in the case of soil.

According to an embodiment, a shape adaptive element is adapted so as to retain the electrolyte volume in the electrolyte reservoir.

For example, a seal on at least one side of the electrode may be formed by adapting the shape adaptive element, such that the electrolyte volume may be at least temporarily retained in the electrolyte reservoir by the shape adaptive element. In particular, the shape adaptive element may be used in combination with a pipe, wherein the shape adaptive element is adapted so as to retain the electrolyte volume in a section of the pipe. As such, the electrolyte reservoir may be at least section-wise delimited by the shape adaptive element or the shape adaptive element in combination with wall sections of a pipe. Particularly, the shape adaptive element may retain the electrolyte volume in the electrolyte reservoir so as to bring it into electrically conductive connection with the material even if the diameter of the pipe varies along the pipe, for example due to limescales, which allows to apply the shape adaptive elements in particular in pipes with various shapes and various diameters.

Preferably, the shape adaptive element is adapted between a first retracted state particularly for allowing simplified positioning of the shape adaptive element and a second extended or expanded state, in which the shape adaptive element is retaining the electrolyte volume in the electrolyte reservoir. This is particularly advantageous in combination with a pipe, wherein the shape adaptive element may be first positioned in the retracted state within the pipe and then extended to the extended state so as to adjust to the inner diameter of the pipe, such that the electrolyte volume is retained in a section of the pipe by the shape adaptive element.

Optionally, the container may comprise an electrolyte supply line for filling the electrolyte reservoir, particularly once the shape adaptive elements are adapted so as to retain the electrolyte volume.

For example, the shape adaptive element may comprise or be an inflatable element configured to be inflated and deflated, wherein particularly in an inflated state of the inflated element, the inflated element is arranged and configured to retain the electrolyte volume in the electrolyte reservoir. As such, the inflated state corresponds to the extended state and the deflated state corresponds to the retracted state of the shape adaptive element.

In another alternative, the shape adaptive element may be realized by a lip seal. Particularly, a diameter of the lip seal may be changed between a first and a second diameter, wherein the second diameter is larger than the first diameter. As such, the second diameter corresponds to the extended state and the first diameter corresponds to the retracted state of the shape adaptive element.

According to another alternative, the shape adaptive element may be realized by an umbrella mechanism allowing for folding and unfolding of a foldable element between a folded and unfolded state, so as to retain the electrolyte volume with the foldable element. For example, the foldable element may be unfolded or folded by inflating or deflating an inflatable element next to the foldable element. Alternatively, the foldable element may be unfolded and folded pneumatically, hydraulically, by electrical- or electromagnetic actuators and/or by a mechanical mechanism such as a gearing.

In yet another alternative, the shape adaptive element is transformed between the retracted and the extended state under plastic deformation of the shape adaptive element. To this end, for example, a force may be conducted via supplementary elements from one or more sides into or against the shape adaptive element, such that the latter transforms into the extended state under plastic deformation. Subsequently the supplementary elements may be removed from the shape adaptive element and the shape adaptive element returns to the retracted state. Preferably, in this alternative, the shape adaptive element comprises or consists of an elastic material such that the plastic deformation is reversible and the shape adaptive element may undergo the plastic deformation reversibly and repeatedly.

Alternatively, the shape adaptive element may be realized by a plurality of flexible bristles providing a seal on at least one side of the electrode.

In another alternative, the shape adaptive element thermally adapts to the extended state under thermal expansion so as to form a seal. Particularly, in this alternative, the shape adaptive element may return to the retracted state under thermal contraction.

Particularly, the shape adaptive elements may be extended by a chemical mechanism induced by a swelling of the shape adaptive element upon contact of the shape adaptive element with a triggering fluid.

According to an embodiment, the electrolyte volume is brought into fluidic connection with the material via at least one opening of the electrolyte reservoir providing an interface between the electrolyte reservoir and the material, such that an electrically conductive contact is realized between the electrolyte volume and the material such that the electrochemical measurement is carried out.

Particularly, if the container is realized by or in combination with a pipe, the at least one opening is a hole or a perforation in the wall of the pipe. In particular drainage lines usually comprise such openings in order to allow seeping water to enter the drainage lines. By retaining the electrolyte reservoir in a section of the pipe comprising at least one opening, particularly using the shape adaptive elements, the electrolyte may flow out of the electrolyte reservoir and into the material through the opening of the pipe. As such, this embodiment takes advantage of existing infrastructure, by using holes or perforations on drainage lines as interfaces between the electrolyte and the material in order so as to perform the electrochemical measurement.

According to another embodiment, the electrolyte is ejected out of the electrolyte volume in form of a jet onto the material, such that an electrically conductive contact is realized between the electrolyte volume and the material via the jet such that the electrochemical measurement is carried out. To this end, the electrolyte is at least temporarily pumped through and out of the electrolyte reservoir in order to maintain the electrical contact with the material via the jet for at least the time of an electrochemical measurement, which may be on the order of a few seconds. As such, the electrolyte reservoir and the material may be brought into electrically conductive contact even if they are spaced from each other, which allows to apply the embodiment in particular in pipes with various shapes and various diameters.

Preferably, if the electrolyte reservoir is arranged in a container, the ejection of the jet takes place via an opening of the container.

According to yet another embodiment, the electrode is moved to multiple locations having respective distances to the metal element and electrochemical measurements are carried out at the multiple locations, such that spatial information indicative of the state of corrosion is obtained in the area of the metal element.

In another embodiment, multiple electrodes are arranged in the electrolyte volume in respective distances to the metal element and multiple electrochemical measurements are carried out with the multiple electrodes, such that the spatial information indicative of the state of corrosion is obtained in the area of the metal element.

Particularly, multiple electrodes may be arranged in the electrolyte volume and the multiple electrodes may be moved to multiple locations, such that the spatial information indicative of the state of corrosion is obtained in the area of the metal element.

For example, an electrode is moved by 15 cm to 100 cm between two subsequent electrochemical measurements or two adjacent electrodes are spaced between 0 cm and 100 cm.

As such, multiple electrochemical measurements may be conducted at multiple locations, which improves the resolution of the actual spatial resolution of the electrochemical environment, such as for example the potential field distribution in the area of the metal element obtained by the multiple electrochemical measurements. Particularly, multiple electrochemical measurements may be conducted at the same time by using multiple electrodes. Alternatively, multiple electrochemical measurements may be performed subsequently, i.e. one after another.

Moreover, the spatial information indicative of the state of corrosion obtained from multiple electrochemical measurements may be used to locate regions of critical states of corrosion by means of a spatial gradient information obtained from determining the spatial gradient of the spatial information. In this representation, regions with substantial variations of the state of corrosions are spatially highlighted, such that areas of the metal element with critical states of corrosions may be efficiently detected.

According to an embodiment of the first aspect of the invention, an electrical resistance between the electrode and the at least one metal element of the concrete structure is determined, particularly prior to the electrochemical measurement, such that an electrical resistance between the electrode and the at least one metal element is tested. If the electrical resistance is higher than for example 100 kΩ as for instance in dry soil or after longer periods with limited precipitation, electrolyte can be injected into the material to make it more electrically conductive.

A second aspect of the invention relates to a device for assessing corrosion phenomena on at least one metal element of a concrete structure covered at least in regions with an material that is electrolytically conductive or can be made electrolytically conductive. The device comprises a container for receiving the electrolyte volume in or next to the material, realizing an electrolyte reservoir, and an electrode arranged or arrangeable in the electrolyte reservoir for forming an electrically conductive connection with the electrolyte. The container has at least one opening for realizing a fluidic connection with the material, thus providing an interface between the electrolyte reservoir and the material such that an electrically conductive contact between the electrode and the material can be realized. The device further comprises a measuring unit for measuring a signal indicative of the state of corrosion of the metal element.

In an embodiment, the device further comprises an electrolyte supply unit, particularly a pump unit, configured to cause a flow of the electrolyte through the electrolyte reservoir and the opening so as to realize an electrically conductive contact between the electrode and the material. To this end, the electrolyte supply unit is preferably in fluidic connection with the electrolyte reservoir via an electrolyte supply line.

According to an embodiment, the device further comprises at least one shape adaptive element arranged and configured to form an expandable and shrinkable seal on at least one side of the electrode, such that the at least one shape adaptive element is configured to retain the electrolyte volume.

For example, the shape adaptive element may be or comprise inflatable elements in fluidic connection with a gas reservoir via a gas line or with a fluid reservoir via a fluid line, such that the inflatable elements may be expanded into an inflated state by conducting pressurized gas from the gas reservoir or a fluid from the fluid reservoir into the inflatable elements.

In particular, in the inflated state, the at least one inflatable element is configured and arranged to form seals on two sides of the electrode, for example if the electrode is arranged in a pipe, such that the electrolyte volume is retained in a section of the pipe. As such, the inflatable element is configured to retain the electrolyte volume on demand in order to carry out an electrochemical measurement at a location along the pipe. Once the electrochemical measurement is finalized, the inflatable elements may be deflated so as to transform to a deflated state by for example releasing the gas, particularly through the gas line, allowing the electrolyte to drain off the electrolyte reservoir.

According to an embodiment, the device may further comprise an electrolyte supply unit configured to cause a flow of electrolyte through the electrolyte reservoir and the opening so as to realize an electrically conductive contact between the electrode and the material via a fluidic connection through the opening.

For example, the electrolyte supply unit may comprise an external source of pressurized electrolyte, such as an external water supply.

In particular, the electrolyte supply unit may comprise a pump unit configured to pump the electrolyte so as to cause said flow of electrolyte.

Particularly, the electrolyte supply unit is configured to cause an ejection of the electrolyte out of the container and through the at least one opening onto the material in form of a jet, such that the fluidic connection with the material may be realized by the jet and the electrically conductive contact may be realized between the electrolyte volume and the material via the jet.

According to yet another embodiment of the second aspect of the invention, the electrode is movable such that the electrode is configured to be positioned in different locations.

Particularly, the container and the electrode are arranged on a movable element, such that by moving the movable element with the container and the electrode to multiple locations in respective distances to the metal element and by carrying out electromagnetic electrochemical measurements at the multiple locations, spatial information indicative of the state of corrosion is obtained in the area of the metal element.

For example, the movable element may be or comprise at least one rolling element and/or at least one sliding element, such that under rolling of the rolling elements and/or under sliding of the sliding elements, the electrode may be moved to different locations.

Particularly, the movable element may be arranged on a seal, particularly a seal formable by the inflatable elements, such the seal may be moved together with the electrode. As such, the electrolyte volume may be retained and sealed together with the pipe on demand at different locations along the pipe, enabling the electrode to be positioned in different locations to perform electrochemical measurements at the different locations.

Alternatively, the container may be or comprise a vessel, wherein the movable elements are mounted on the vessel, allowing translational movement of the vessel. As such, the vessel may be moved to different locations, particularly different locations along a pipe. Preferably, if the container is or comprises a vessel, the electrically conductive connection between the electrolyte reservoir and the material is realized via the jet.

In another embodiment, the device further comprises multiple electrodes in respective distances to the metal element, such that by carrying out electrochemical measurements with the multiple electrodes, the spatial information indicative of the state of corrosion is obtained in the area of the metal element.

Particularly, the device according to the second aspect of the invention or one of its embodiments is configured to perform the method according to the first aspect of the invention or one of its embodiments.

Exemplary embodiments are described below in conjunction with the Figures. The Figures are appended to the claims and are accompanied by text explaining individual features of the shown embodiments and aspects of the present invention. Each individual feature shown in the Figures and/or mentioned in the text of the Figures may be incorporated (also in an isolated fashion) into a claim relating to the method and device for assessing corrosion phenomena according to the present invention.
- Fig. 1: shows a cantilever retaining wall comprising a metal element, wherein the cantilever retaining wall secures an embarkment;
- Fig. 2: shows a method for assessing corrosion phenomena according to the prior art, wherein a core sample is taken out of the retaining wall so as to assess the corrosion of the metal element;
- Fig. 3: shows a method for assessing corrosion phenomena according to the prior art, wherein the cantilever retaining wall is partially removed in order to create an inspection window for assessment of the corrosion of the metal element from a front side of the cantilever retaining wall;
- Fig. 4: shows a method for assessing corrosion phenomena according to the prior art, wherein an inspection shaft is excavated at a back side of the cantilever retaining wall facing the embarkment for assessing the corrosion on the metal element;
- Fig. 5: shows an exemplary embodiment for a method and a device for assessing corrosion phenomena in a cantilever retaining wall according to the invention;
- Fig. 6: sketches a method used for a potential field measurement for assessment of corrosion phenomena in the prior art;
- Fig. 7: illustrates a potential field distribution around a metal element measured using multiple electrodes arranged along the metal element;
- Fig. 8: shows a method and device for assessing corrosion phenomena in accordance with an embodiment of the invention;
- Fig. 9: highlights the electrolyte volume arranged in a container introduced in Fig. 8, wherein the electrolyte volume is retained using inflatable elements which are shown in an inflated state;
- Fig. 10: highlights the inflatable elements of Fig. 9 in a deflated state and
- Fig. 11: shows another embodiment of a method and device in accordance with the invention, wherein the electrically conductive contact between the electrolyte volume and the material is realized by a jet ejected out of a container.

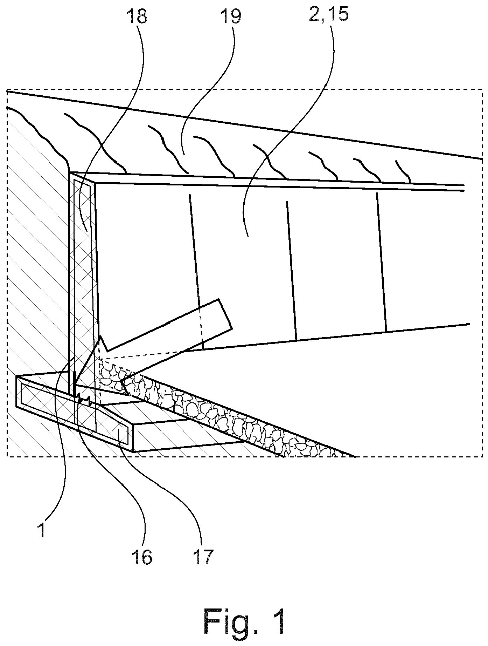

Fig. 1 shows a concrete structure 2, in particular a cantilever retaining wall 15 for securing an embankment 19. Cantilever retaining walls 15 are reinforced concrete structures 2 which usually comprise a foundation slab 17 and a stem 18 connected by a construction joint 16. The slope pressure on the stem 18 causes a bending moment on the construction joint 16 between the foundation slab 17 and the stem 18. This bending moment causes high tensile forces on the construction joint 16. Concrete has a high compressive strength but a statically negligible tensile strength, this tensile strength is ensured or taken over by metal elements 1, particularly steel elements forming a reinforcement. Due to the high tensile forces on the slope side between the foundation slab 17 and the stem 18, an intact metal element 1, particularly steel element in the area above of the construction joint 16 is essential for the load-bearing capacity of the cantilever retaining wall 15. This section of the metal element 1, particularly steel element has been indicated in Fig. 1 with the arrow and additionally highlighted with a thicker line, as it is of special interest for the inspection of cantilever retaining walls 15. As can be seen here, the metal element 1 in this example forms a network of multiple interconnected sections extending both vertically and horizontally in the shown cross-section and extends along the entire length of the cantilever retaining wall 15.

However, the metal element 1 is particularly vulnerable to corrosion phenomena, which deteriorate its structural stability and as such the stability of the cantilever retaining wall 15. At the same time, the construction joint 16 and particularly the metal element 1 in the area above of the construction joint 16 are subject to high forces, such that advanced corrosion phenomena on the metal element 1 may ultimately result in unannounced collapses of the cantilever retaining wall 15. It is therefore necessary to regularly assess the state of corrosion on the metal element 1 in the area above of the construction joint 16 in order to reduce the risk of collapses, which represent a source of danger for people and cause high costs.

Fig. 2 sketches a method for assessing corrosion phenomena according to the prior art. In this method, core drillings are used to locally extract core samples of the metal element 1 in the area above of the construction joint 16 by drilling a drill hole 21 through the cantilever retaining wall 15 in order to assess the metal element 1 in the area of the construction joint 16. The core sample is then analyzed regarding its state of corrosion. This method is inconvenient at least because of the following. First, the drilling of drill holes 21 to extract core samples represents an invasive method that affects the stability of the cantilever retaining wall 15. Second, this method only allows to locally, that is at the location of the drilling, assess the state of corrosion on the metal element 1. The drill holes 21 and the extracted core samples typically have a diameter of around 30 cm, such that the assessment of the state of corrosion is constricted to this order of magnitude for each sample according to this method. As a consequence, potentially dangerous corrosion phenomena in the proximity of or generally outside the drill holes 21 selected for extraction of core samples cannot be assessed using this method. Moreover, the more locations are chosen for drillings to generate a more accurate picture of the state of corrosion on the metal element 1, the more the stability of the cantilever retaining wall 15 is harmed by this destructive method. Third, the analysis of the extracted sample involves further steps, such as separation of the metal element 1 from concrete material and for example a visual analysis.

Fig. 3 illustrates another method for assessing corrosion phenomena according to the prior art. According to this method, the concrete of the cantilever retaining wall 15, particularly its stem 18, is partially removed in order to create an inspection window 20 for inspection of the metal element 1 from a front side of the cantilever retaining wall 15 facing away from the embarkment 19. The concrete may thereby for example be removed by high water pressure or other techniques known from the prior art. The inspection window 20 may extend several meters along the metal element 1, such that the state of corrosion may be assessed along the entire extent of the inspection window 20 with this method. Nevertheless, this method is still inconvenient as it also represents a destructive method that may itself cause a loss of stability of the cantilever retaining wall 15 due to the removal of concrete, particularly in the area of the construction joint 16. Moreover, while the inspection window 20 allows for a larger region of the metal element 1 to be analyzed regarding its state of corrosion compared to the method depicted in Fig. 2, it is still constricted to the extent of the inspection window 20, such that the state of corrosion of the metal element 1 may not be assessed outside the inspection window 20. As such, potentially dangerous corrosion phenomena outside of the inspection window 20 selected for inspection cannot be assessed using this method.

Fig. 4 depicts yet another method for assessing corrosion phenomena according to the prior art. Here, an inspection shaft 22 is excavated at a back side of the cantilever retaining wall 15 facing the embarkment 19 in order to inspect the metal element 1. As such, this method permits to assess the state of corrosion on the metal element 1 along the extent of the inspection shaft 22 while ideally keeping the structure of the cantilever retaining wall 15 near the inspection shaft 22 intact. However, this method requires the excavation of large quantities of soil and/or gravel comprised by the embarkment 19, making the method complex and expensive. At the same time, the assessment of the state of corrosion remains constricted to the region of the excavated inspection shaft 22, such that potentially dangerous corrosion phenomena outside the excavation shaft 22 selected for inspection cannot be assessed using this method.

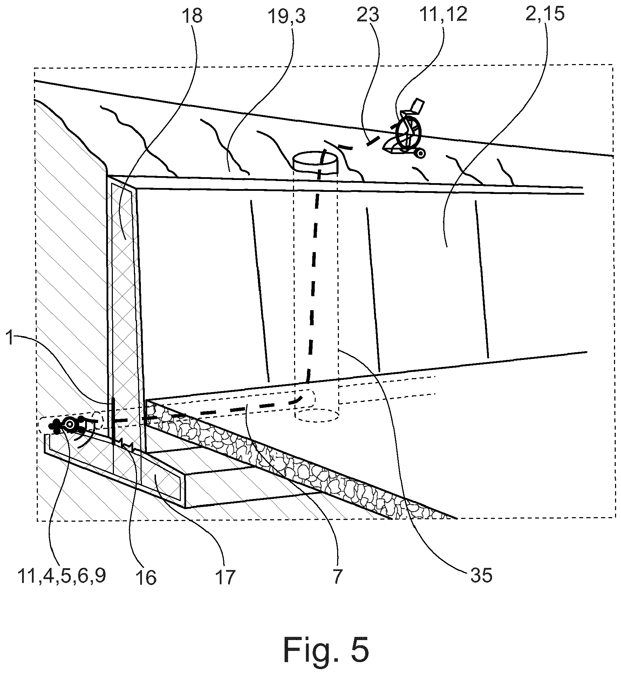

Fig. 5 shows an exemplary embodiment for a method and a device 11 for assessing corrosion phenomena according to the invention. According to the first aspect of the invention, an electrolyte volume 4 is positioned in a material 3, an electrode 5 is arranged in the electrolyte volume 4 so that the electrode 5 and the electrolyte are in electrically conductive connection, and the electrolyte volume 4 is brought into electrically conductive contact with the material 3, and an electrochemical measurement indicative of a state of corrosion of the metal element 1 is carried out.

As indicated in Fig. 5, the material 3 may be realized by the embarkment 19, particularly soil and/or gravel of the embarkment arranged between the electrode 4 and the metal element 1. As such, the state of corrosion of the metal element 1 may be assessed in a non-invasive manner, leaving the structure of the cantilever retaining wall 15 intact.

The embodiment shown in Fig. 5 further takes advantage of a pipe 7 spaced from the metal element 1 and extending parallel along the cantilever retaining wall 15. Such a pipe 7 is often built together with the cantilever retaining wall 15, forming a drainage line that serves as a drain for liquids, particularly water, diffusing through the soil of the embarkment 19. The pipe 7 may be accessed via the embarkment 19 by existing pipe systems, such as maintenance shafts 35 and the like, a drill hole 21 or an excavation.

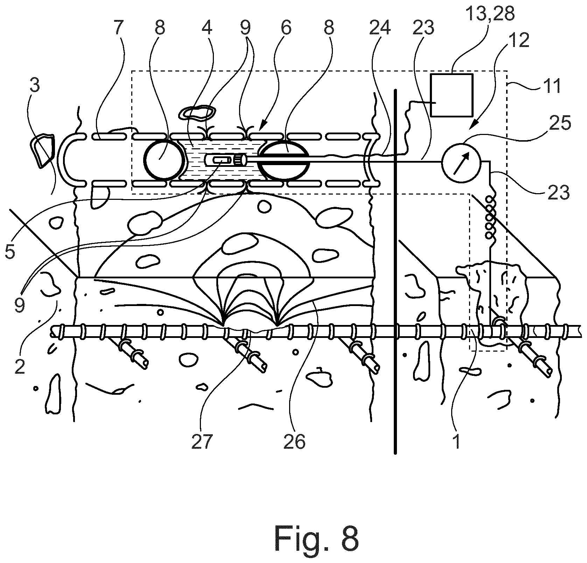

The device 11 according to the invention may be arranged at least partially inside the pipe 7 to perform the method according to the first aspect of the invention. According to the embodiment shown in Fig. 5, a container 6 for receiving the electrolyte volume 4 in or next to the material 3, realizing an electrolyte reservoir, and the electrode 5 arranged or arrangeable in the electrolyte reservoir for forming an electrically conductive connection with the electrolyte are arranged inside the pipe 7. The container 6 has at least one opening 9 for realizing a fluidic connection with the material 3, such that the opening 9 provides an interface between the electrolyte reservoir and the material 3, such that an electrically conductive contact between the electrode 5 and the material 3 can be realized. Particularly, as highlighted in Fig. 8 to Fig. 11, the electrically conductive contact between the electrode 5 and the material 3 may be realized by openings 9 of the pipe 7, such that the pipe becomes part of the container 6. The device 11 further comprises a measuring unit 12 for measuring a signal indicative of the state of corrosion on the metal element 1. To this end, the measuring unit 12 is electrically connected to the electrode 5 via an electrical conductor 23 such as a wire or a cable. The electrical circuit of the measuring unit 12 is further depicted in Fig. 8.

The electrode 5, particularly the electrode 5 and the container 6 for receiving the electrolyte volume 4 may be moved to multiple different locations along the pipe 7 and perform electrochemical measurements at these locations, such that the state of corrosion can be assessed at the different locations. For example, the electrochemical measurements may comprise at least one of the following: potential measurements, particularly potential field measurements; electrochemical impedance measurements; gavanostatic- or potentiostatic polarization-, galvanodynamic- or potentiodynamic polarization measurements, particularly for instance linear polarization resistance measurements; Harmonic analysis or Faradaic rectification measurements. Particularly, two locations at which successive electrochemical measurements are carried out are spaced less than a characteristic distance. The characteristic distance is preferably chosen to be less than 1 m, which is smaller than the length scale over which the state of corrosion is known to vary substantially. As such, the invention advantageously permits to assess the state of corrosion of the metal element 1 in a quasi-continuous manner, such that no gaps of unknown, potentially dangerous states of corrosion of the metal element 1 remain while keeping a measurement time and thus the effort and costs at a minimum.

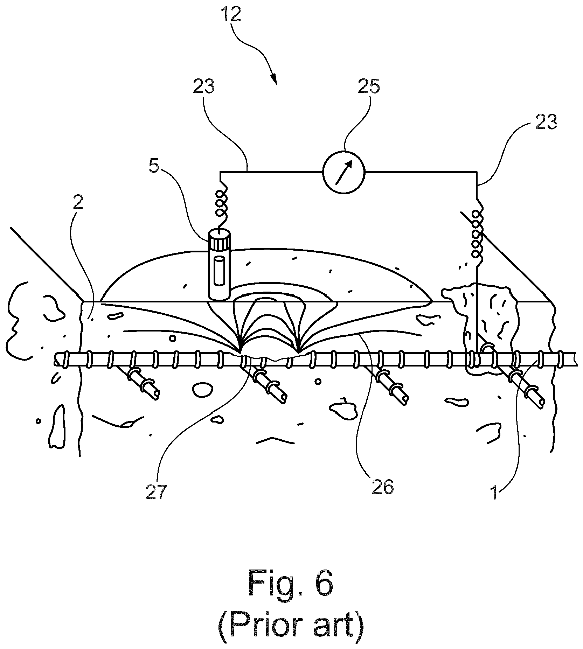

Fig. 6 sketches a method and device used for a potential field measurement for assessment of corrosion phenomena in the prior art. The potential field measurement is carried out with a measuring unit 12 comprising an electrode 5, a measuring device 25 and electrical conductors 23. To this end, the measuring device 25 is electrically connected to the electrode 5 and to the metal element 1 via electrical conductors 23, such that an electrical potential difference and/or an electrical voltage between the electrode 5 and the metal element 1 may be measured using the measuring unit 12.

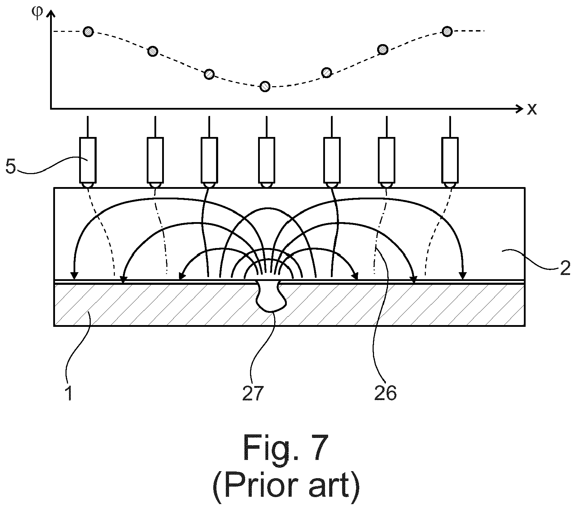

In principle, the metal element 1 is in a passive state due to the surrounding alkaline concrete structure 2, which reduces the corrosion rate to a level that is no longer technically relevant. However, the passivity of the metal element 1 can be cancelled by various mechanisms, which leads to a significant increase of the state of corrosion over time. In the case of concrete structures 2 reinforced by metal element 1, particularly cantilever retaining walls 15, in particular the following three main corrosion mechanisms can be distinguished: corrosion by chlorides, corrosion by carbonation, corrosion due to insufficient concrete coating. For the structural safety of cantilever retaining walls 15, corrosion by chlorides and by insufficient concrete coating are the most important. Corrosion damages caused by chloride ingress, for example from de-icing agents from roads on the embarkment 19 adjacent to the cantilever retaining wall 15 and structural deficits, such as cracks and/or voids can therefore occur heterogeneously along the cantilever retaining wall 15. As an example, the metal element 1 sketched in Fig. 6 comprises a corrosion region 27 with an advanced state of corrosion. Due to its electrochemical nature, the corrosion goes along with a migration of ions and electrons, causing different electric potentials in regions of accumulations of opposite charges. In particular in case of corrosion related to chlorides, the corresponding electrical potential differences between anodic corrosion regions 27 and cathodic regions of the metal element 1 not affected by corrosion may correspond to voltages of several hundred mV. If the electrical connection between the metal element 1 and the measuring device 25 is connected at a region of the metal element 1 which is essentially free of corrosion, the measured potential differences or voltages indicate deviations from a metal element 1 with corrosion. As such, larger absolute values of the potential differences or voltages correspond to a stronger state of corrosion and vice versa. The potential differences are thus indicative for the state of corrosion and can be measured using conventional electronic measurement equipment or -devices. The three-dimensional spatial distribution of the potential field around the corrosion region 27 shown in Fig. 6 and Fig. 7 is indicated with isopotential lines 26.

To assess the state of corrosion in the corrosion region 27 using the method and device according to the prior art depicted in Fig. 6, the three-dimensional potential field is probed with the electrode 5. To this end, according to the prior art, the electrode 5 is arranged at a location on the concrete structure 2. Now, the potential field measurement is carried out by means of a potential difference or voltage measurement, determining the potential difference or voltage between the electrode 5 and the metal element 1 at the location of the electrode 5. A strength of the potential difference or voltage measured at this particular location, corresponds to a single value of the potential field at a three-dimensional coordinate of the three-dimensional potential field distribution in the area of the metal element 1. By repeating the procedure at different locations, more and more information about the actual three-dimensional potential distribution is gained, such that corrosion phenomena, particularly corrosion regions 27 on the metal element 1 may be assessed and detected.

This principle is further illustrated in Fig. 7. Here, a concrete structure 2 reinforced with a metal element 1, particularly a steel element is sketched, wherein the potential field distribution around the metal element 1 is assessed by arranging multiple electrodes 5 on the concrete structure 2, according to the prior art. As the multiple electrodes 5 are each placed at a different location, they each probe a respective value of the potential field at their respective location. The respective value of the potential field cp measured by the respective electrode is plotted as a function of the location x along the metal element 1 in Fig. 7, indicated as dots. If the spacing between adjacent electrodes 5 is chosen small enough, such that the state of corrosion does not deviate substantially from the known, measured value at one of the locations over the spacing, the unknown potential field values between the measured values may be estimated by a smooth interpolation between the measured dots, indicated as a dashed line.

Fig. 8 illustrates a method and device 11 for assessing corrosion phenomena in accordance with an embodiment of the invention allowing to assess the corrosion on a metal element 1 of a concrete structure 2 covered at least partially with a material 3 that is electrolytically conductive or can be made electrolytically conductive. The material may for example be soil or gravel, such that the concrete structure 2, for example a cantilever retaining wall 15, is in electrolytic contact with the soil or gravel. Particularly in the case of soil, due to the moisture in the soil, the potential field of the corrosion reaction spreads not only in the concrete structure 2 but also in the material 3, that is the soil. Similarly, for example in the case of gravel, gaps between particles forming the gravel allow for an introduction of the electrolyte into the gravel, making the material 3 electrolytically conductive. Thus, direct contact of the electrode 5 with the concrete structure 2 is not necessary for an electrochemical measurement as is the case of concrete structures 2 according to the prior art, cf. Fig. 6 and Fig. 7. A complex excavation of large amounts of soil or drilling holes that may damage the concrete structure 2, as required in the prior art, is therefore no longer necessary. The embodiment shown here in Fig. 8 takes advantage of a pipe 7, particularly a drainage line, which is often present parallel to cantilever retaining walls 15. Normally, the drainage lines are made of plastic or, in the case of earlier structures, of concrete and have openings 9 or perforations at the top through which seeping soil water may enter the drainage lines so as to drain off.

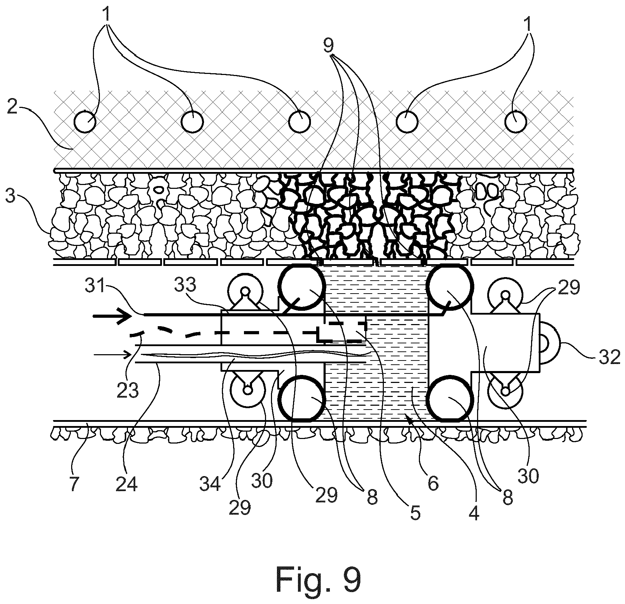

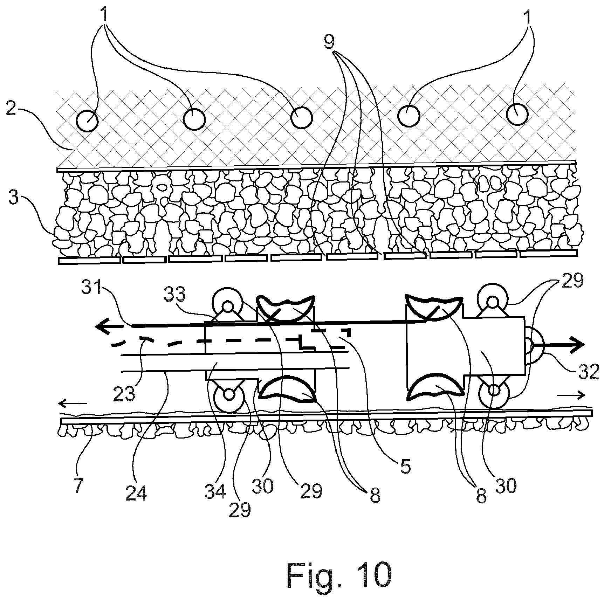

In further contrast to the prior art, the electrode 5 of the measuring unit 12 is arranged in an electrolyte volume 4 arranged in a container 6. The container 6 is formed in a pipe 7, for example a drainage line, and realized particularly by shape adaptive elements 8 forming seals on two sides of the electrode 5, thereby retaining the electrolyte volume 4 in a section of the pipe 7, as explained in more detail in Fig. 9 and Fig. 10.

As such, the electrode 5 in the container 6 is separated from the concrete structure 2 by the material 3, i.e. the soil between the concrete structure 2. The electrolyte is pumped by an electrolyte supply unit 13, particularly a pump unit, from an electrolyte supply 28 through an electrolyte supply line 24 into the container 6. The electrolyte preferably comprises or consists of an aqueous solution with adequate ionic strength, for example tap water, wherein a sodium sulfate or another salt may be added. The electrolyte seeps out of the electrolyte volume 4 via the openings 9 of the container 6, which are here realized by openings 9 of the pipe 7 forming interfaces between the electrolyte volume 4 and the material 3. Since such openings 9 are already present especially on drainage lines, the method and the device 11 according to this embodiment can be used advantageously on already existing infrastructure. Via the openings 9, the electrode 5 arranged in the electrolyte volume 4 is brought into electrically conductive contact with the material 3, such that the electrochemical measurement can be carried out. As such, the invention permits to assess corrosion phenomena on metal elements 1 of concrete structures 2 covered at least partially with a material 3 that is electrolytically conductive or can be made electrolytically conductive.

Fig. 9 highlights the electrolyte volume 4 arranged in a container 6 introduced in Fig. 8, which is part of the device 11 in accordance with an embodiment of the invention. The container 6 arranged in a pipe 7, particularly a drainage line, is shown in a cut view along the pipe 7, such that the electrolyte in the electrolyte volume 4 can be seen in the container 6.

According to this embodiment, the container 6 is formed by shape adaptive elements 8 arranged on a base element 30, which also serves as a suspension for rolling elements 29, as will be further addressed in Fig. 10 below. The shape adaptive elements 8 in Fig. 9 and Fig. 10 are, by means of an example, represented by inflatable elements. However, the shape adaptive elements 8 may also be realized by, for example, a lip seal, an umbrella mechanism, a plastically deformable element, flexible bristles and/or thermally or chemically deformable elements. In case of inflatable shape adaptive elements 8, they may be inflated by pressurized gas, for example air, conducted into the inflatable elements via a gas line 31. To this end, the gas line 31 may be guided through the base element 30 via a gas grommet 33, such that the gas can be conducted into the inflatable elements. The gas line 31 may be guided through the pipe 7, such that it can be fluidically connected to a gas source and particularly a compressor for supplying the inflatable elements with pressurized gas. As can further be seen in Fig. 9, the base element 30 serves as a mount for the electrode 5, such that the electrode may be kept at a fixed location for an electrochemical measurement.

In an inflated state of the inflatable shape adaptive elements 8, which is shown here in Fig. 9, the inflatable shape adaptive elements 8 are expanded by pressurized gas and form seals on two sides of the electrode 5 along the pipe 7, such that the electrolyte volume 4 is retained in the container 6 thus formed between the inflatable shape adaptive elements 8 and the pipe 7. The pipe 7 in this example embodiment comprises openings 9 or perforations which thereby realize openings 9 of the container 6, such that the electrolyte may flow into the material 3 between the electrolyte volume 4 and the concrete structure 2. As such, openings 9 provide an interface between the electrolyte reservoir 4 and the material 3, such that an electrically conductive contact between the electrode 5 and the material 3 can be realized, thereby enabling the electrochemical measurement. In this embodiment, the opening 9 forms a direct interface between the electrolyte volume 4 and the material 3, bringing the two into electrically conductive contact.

For the electrochemical measurement, the electrolyte does not need to reach all the way down to the level of the concrete structure 2. However, the electrode 4 needs to be in electrically conductive contact with the material 3. To maintain this electrically conductive contact despite the permanent drain or seepage of electrolyte into the soil for at least the time period of a single electrochemical measurement at one location of the electrode 5, which may be on the order of a few seconds, the electrolyte is pumped through the container 6 via an electrolyte supply line 24 for at least this time period. As such, the electrolyte supply line 24 may form a section of the container 6. To this end, the base element 30 comprises an electrolyte grommet 34 for conducting the electrolyte through the electrolyte supply line 24 into and through the container 6.

Once the electrochemical measurement is performed at a selected location along the pipe 7, the electrode 5 may be moved to another location along the pipe 7 so as to perform another electrochemical measurement to obtain information indicative of the state of corrosion at the other location. To this end, the inflatable shape adaptive elements 8 may be deflated by releasing the pressurized gas from the inflatable shape adaptive elements 8, such that they transform to a deflated state, which is depicted in Fig. 10.

The deflation or shrinking of the inflatable shape adaptive elements 8 causes i) a draining of the electrolyte volume 4 out of the container 6, wherein the electrolyte flows away from the electrode 5 along the two directions of the pipe 7 and ii) a touchdown of the lower two rolling elements 29 suspended on the base element 30, viewed in the plane of the drawing. Now, the electrode 5 may be moved to another location along the pipe 7 via the rolling elements 29, such that another electrochemical measurement may be carried out at the other location, whereby the inflatable shape adaptive elements 8 are again inflated to the inflated state shown in Fig. 9. To this end, the device 11 to this embodiment comprises a hook 32 protruding from the base element 30, which may be used to pull the electrode 4 on the base element 30 by a wire or a rope. Alternatively, the device 1 may be equipped with a push rod, such that the electrode 4 on the base element 30 may be pushed to different locations along the pipe 7 via the push rod. In yet another alternative, the device may comprise an electrical actuator powered with a battery or an electronic power supply cable for driving the rolling elements 29 or caterpillar drive, such that the electrode 4 on the base element 30 may move along the pipe 7 powered by the actuator.

The inflatable shape adaptive elements 8 may be repeatedly inflated, deflated and moved to another position along the pipe 7, such that multiple electrochemical measurements may be performed, allowing to obtain spatial information indicative of the state of corrosion of the metal element 1 in the area above of the construction joint 16. This procedure allows a discrete, but compared to the length of a cantilever retaining wall practically continuous electrochemical measurement at locations along the length of the pipe 7 or drainage line, such that for instance an entire cantilever retaining wall 15 comprising a pipe 7 drainage line in its vicinity may be assessed regarding the state of corrosion of the metal elements 1 in the area above of the construction joint 16 of the cantilever retaining wall 15.

As can be understood from comparing Fig. 9 and Fig. 10, the rolling elements 29 and the inflatable shape adaptive elements 8 are configured and arranged such on the base element 30, that in the inflated state of the inflatable shape adaptive elements 8 shown in Fig. 9, the inflatable shape adaptive elements 8 are in contact with the pipe 7 while the rolling elements 29 are not and that in the deflated state of the inflatable shape adaptive elements 8 shown in Fig. 10, the rolling elements 29 are in contact with the pipe 7 while the inflatable shape adaptive elements 8 are not. As such, the electrode 5 may on the one hand be fixedly kept at a location along the pipe for an electrochemical measurement, friction-locking the inflatable shape adaptive elements 8 with the pipe 7 in the inflated state with the rolling elements 29 spaced from the pipe 7. The method and the device 11 according to the present embodiment may therefore also be used in pipes 7 comprising a slope resulting in a force that could cause the electrode 5 on the base element 30 to move along the pipe 7 without the inflatable shape adaptive elements 8. On the other hand, this arrangement of the rolling elements 29 and the inflatable shape adaptive elements 8 allows to move the electrode 5 to different locations along the pipe 7 between two electrochemical measurements via the rolling elements 29 in the deflated state.

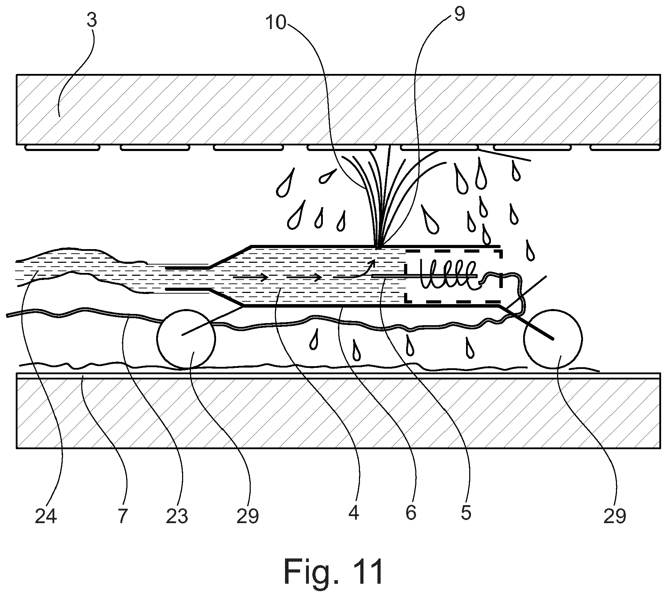

Fig. 11 shows another embodiment for a method and device 11 in accordance with the invention. The device 11 comprises a container 6 for receiving the electrolyte volume 4, wherein the container 6 is arranged in a pipe 7, particularly a drainage line. The electrode 5 is arranged in the container 6, such that it is in electrical connection with the electrolyte volume 4 once the container 6 is filled with electrolyte, as it is the case in Fig. 10. The container 6 is in fluidic connection with an electrolyte supply 28 (not shown) via an electrolyte supply line 24 and further comprises an opening 9. The electrolyte is pumped through the container 6 and the opening 9, such that the electrolyte is ejected out of the electrolyte volume 4 in form of a jet 10 onto the material 3, realizing an electrically conductive contact between the electrolyte volume 4 and the material 3 via the jet 10, such that the electrochemical measurement may be carried out at a location along the pipe 7. In this embodiment, the opening 9 forms an intermediate interface between the electrolyte volume 4 and the material 3, which are ultimately brought into electrically conductive contact via the jet 10 formed between the opening 9 and the material 3.

Particularly, the electrolyte is pumped for at least the time period of a single electrochemical measurement at a first location so as to maintain the electrically conductive contact between the electrode 5 and the material 3 for the time period of the electrochemical measurement. Once the electrochemical measurement at the first location is finalized, the pumping of the electrolyte causing the ejection of the jet 10 may be stopped such that no electrolyte is wasted. Now, the electrode 5 and the container 6 may be moved, particularly pulled or pushed, along the pipe 7 to a second location away from the first location using the rolling elements 29 suspended on the container 6. At the second location, the pumping may be restarted for another electrochemical measurement at the second location. The process of pumping, stopping the pumping and moving to another location may be repeated multiple times, such that the information indicative for the state of corrosion in the area of the metal element 1 (not shown) near the material 3 is obtained.

Alternatively, the electrochemical measurement is conducted wherein the electrolyte is pumped continuously, particularly wherein the electrode and the container are moved continuously, i.e. without stopping at particular locations. In case the jet 10 does not form a constant electrically conductive contact with the material 3 during the time of a electrochemical measurement, data corresponding to measured electrochemical quantities indicative for the state of corrosion may be digitally processed or corrected in a post-processing procedure, wherein for example erroneous data obtained while no or no sufficient electrically conductive contact was established that deviate from a predetermined or measured average value of the electrostatic potential value are sorted out and not taken into account for assessing the state of corrosion.

**List of reference signs**

| | |
|---|---|
| Metal element | 1 |
| Concrete structure | 2 |
| Material | 3 |
| Electrolyte volume | 4 |
| Electrode | 5 |
| Container | 6 |
| Pipe | 7 |
| Shape adaptive element | 8 |
| Opening | 9 |
| Jet | 10 |
| Device | 11 |
| measuring unit | 12 |
| Electrolyte supply unit | 13 |
| Cantilever retaining wall | 15 |
| Construction joint | 16 |
| Foundation slab | 17 |
| Stem | 18 |
| Embarkment | 19 |
| Inspection window | 20 |
| Drill hole | 21 |
| Inspection shaft | 22 |
| Electrical conductor | 23 |
| Electrolyte supply line | 24 |
| Measuring device | 25 |
| Isopotential line | 26 |
| Corrosion region | 27 |
| Electrolyte supply | 28 |
| Rolling element | 29 |
| Basis element | 30 |
| Gas line | 31 |
| Hook | 32 |
| Gas grommet | 33 |
| Electrolyte grommet | 34 |
| Maintenance shaft | 35 |

## Claims

1. Method for assessing corrosion phenomena on at least one metal element (1) of a concrete structure (2) covered at least in regions with a material (3) that is electrolytically conductive or can be made electrolytically conductive, in which an electrolyte volume (4) is positioned within the material (3), an electrode (5) is arranged in the electrolyte volume (4) so that the electrode (5) and the electrolyte are in electrically conductive connection, and the electrolyte volume (4) is brought into electrically conductive contact with the material (3), and an electrochemical measurement indicative of a state of corrosion on the metal element (1) is carried out.

2. The method for assessing corrosion phenomena according to claim 1, wherein the electrolyte volume (4) is placed in a container (6) for receiving the electrolyte volume (4) in or next to the material (3), realizing an electrolyte reservoir.

3. The method for assessing corrosion phenomena according to claim 2, wherein the container (6) is at least a part of a pipe (7).

4. The method for assessing corrosion phenomena according to one of the claims 2 or 3, wherein a shape adaptive element (8) is adapted so as to retain the electrolyte volume (4) in the electrolyte reservoir.

5. The method for assessing corrosion phenomena according to one of the claims 2 to 4, wherein the electrolyte volume (4) is brought into fluidic connection with the material (3) via at least one opening (9) of the electrolyte reservoir providing an interface between the electrolyte reservoir and the material (3), such that an electrically conductive contact is realized between the electrolyte volume (4) and the material (3) such that the electrochemical measurement is carried out.

6. The method for assessing corrosion phenomena according to one of the preceding claims, wherein the electrolyte is ejected out of the electrolyte volume (4) in form of a jet (10) onto the material (3), such that an electrically conductive contact is realized between the electrolyte volume (4) and the material (3) via the jet (10) such that the electrochemical measurement is carried out.

7. The method for assessing corrosion phenomena according to one of the preceding claims, wherein the electrode (5) is moved to multiple locations having respective distances to the metal element (1) and wherein electrochemical measurements are carried out at the multiple locations, such that spatial information indicative of the state of corrosion is obtained in the area of the metal element (1).

8. The method for assessing corrosion phenomena according to one of the preceding claims, wherein multiple electrodes (5) are arranged in the electrolyte volume (4) in respective distances to the metal element (1) and multiple electrochemical measurements are carried out with the multiple electrodes (5), such that the spatial information indicative of the state of corrosion is obtained in the area of the metal element (1).

9. The method for assessing corrosion phenomena according to one of the preceding claims, wherein an electrical resistance between the electrode (5) and the at least one metal element (1) is determined, particularly prior to the electrochemical measurement, such that an electrical conductivity between the electrode (5) and the at least one metal element (1) is tested.

10. Device (11) for assessing corrosion phenomena on at least one metal element (1) of a concrete structure (2) covered at least in regions with a material (3) that is electrolytically conductive or can be made electrolytically conductive, comprising a container (6) for receiving the electrolyte volume (4) in or next to the material (3), realizing an electrolyte reservoir, an electrode (5) arranged or arrangeable in the electrolyte reservoir for forming an electrically conductive connection with the electrolyte, wherein the container (6) has at least one opening (9) for realizing a fluidic connection with the material (3) and thus providing an interface between the electrolyte reservoir and the material (3) such that an electrically conductive contact between the electrode (5) and the material (3) can be realized, and wherein the device (11) comprises a measuring unit (12) for measuring a signal indicative of the state of corrosion on the metal element (1).

11. The device (11) for assessing corrosion phenomena according to claim 10, wherein the device (11) comprises at least one shape adaptive element (8) arranged and configured to form an expandable and shrinkable seal on at least one side of the electrode (5), such that the at least one shape adaptive element (8) is configured to retain the electrolyte volume (4).

12. The device (11) for assessing corrosion phenomena according to claim 10 or 11, further comprising an electrolyte supply unit (13) configured to cause an ejection of the electrolyte out of the container (6) and through at least one opening (9) onto the material (3) in form of a jet (10), such that the fluidic connection with the material (3) may be realized by the jet (10) and the electrically conductive contact may be realized between the electrolyte volume (4) and the material (3) via the jet (10).

13. The device (11) for assessing corrosion phenomena according to one of the claims 10 to 12, wherein the electrode (5) is movable such that the electrode (5) is configured to be positioned in different locations.

14. The device (11) for assessing corrosion phenomena according to claim 13, wherein the container (6) and the electrode (5) are arranged on a movable element, such that by moving the movable element with the container (6) and the electrode (5) to multiple locations in respective distances to the metal element (1) and by carrying out electrochemical measurements at the multiple locations, spatial information indicative of the state of corrosion is obtained in the area of the metal element (1).

15. The device (11) for assessing corrosion phenomena according to one of the claims 10 to 14, comprising multiple electrodes (5) in respective distances to the metal element (1), such that by carrying out electrochemical measurements with the multiple electrodes (5), the spatial information indicative of the state of corrosion is obtained in the area of the metal element (1).
